(19) Europäisches Patentamt
European Patent Office
Office européen des brevets

(11) Veröffentlichungsnummer: **0 021 004**
**B1**

(12) # EUROPÄISCHE PATENTSCHRIFT

(45) Veröffentlichungstag der Patentschrift: **04.08.82**

(51) Int. Cl.³: **A 61 L 15/07**, C 08 L 75/12 // C08K5/34, C08K5/20

(21) Anmeldenummer: **80102683.2**

(22) Anmeldetag: **14.05.80**

(54) **Selbsthärtendes Material zur Herstellung eines witterungsbeständigen, nicht vergilbenden Stützverbands.**

(30) Priorität: **25.05.79 DE 2921163**

(43) Veröffentlichungstag der Anmeldung: **07.01.81 Patentblatt 81/1**

(45) Bekanntmachung des Hinweises auf die Patenterteilung: **04.08.82 Patentblatt 82/31**

(84) Benannte Vertragsstaaten: **AT BE CH DE FR GB IT LI NL SE**

(56) Entgegenhaltungen:
**CH-A-478 878**
**DE-A-2 737 670**
**DE-A-2 737 671**
**FR-A-2 369 830**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Reichmann, Wolfgang, Dr., Vohwinkelallee 19, D-4000 Düsseldorf (DE)**
Erfinder: **Lehnert, Günther, Dr. c/o Cutter Laboratories Inc., Fourth & Parker Streets, Berkely Cal. 94710 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Selbsthärtendes Material zur Herstellung eines witterungsbeständigen, nicht vergilbenden Stützverbands

Die Verwendung von mit Gips imprägnierten Binden als versteifendes Verbandsmaterial ist bekannt. Derartige Gipsverbände vergilben nicht, jedoch sind sie unterwünscht schwer, wenig luftdurchlässig, verlieren in feuchtem Zustand, beispielsweise bei der Einwirkung von Wasser auf den ausgehärteten Verband, rasch an Festigkeit, sie behindern wegen ihrer Röntgenabsorption und -streuung die diagnostische Auswertung von Röntgenaufnahmen und sind wegen ihrer mangelhaften Wasserfestigkeit oft Anlaß zu Hautirritationen, hervorgerufen durch Bakterien- oder Pilzbewuchs im Verband.

Es mangelte daher nicht an Versuchen, Verbandmaterialien zur Verfügung zu stellen, die nicht mit diesen Nachteilen behaftet sind. So wurde beispielsweise bereits versucht, Verbandmaterial mit durch UV-Licht härtbaren Polymerlösungen zu tränken und den damit hergestellten Verband durch Bestrahlung mit einer UV-Lampe zu härten (Chemical Orthopaedics and Related Research 103, 109—117 [1974]).

Der hierbei erforderliche Umgang mit UV-Strahlern ist umständlich; auch erreicht das UV-Licht nur die oberen Schichten des Verbands, so daß eine Härtung in tieferen Lagen gar nicht erfolgt oder längere Zeit erfordert. Ein weiterer gravierender Nachteil dieses Verfahrens ist in dem Umstand begründet, daß während des Aushärtens durch UV-Bestrahlung eine Beobachtung der Bruchstelle durch Röntgenkontrolle nicht möglich ist.

In der DE-OS 2 353 212 wird ein versteifendes Verbandmaterial beschrieben, welches aus einem flexiblen Grundmaterial besteht, das mit Oxicarbonylisocyanatgruppen enthaltenden Substanzen ausgerüstet ist. Das Verbandmaterial der DE-OS 2 353 212 konnte sich in der Praxis jedoch nicht durchsetzen, weil einerseits die Herstellung der Binden wegen der extremen Reaktivität der Oxicarbonylisocyanate auf kaum überwindbare Schwierigkeiten stieß und andererseits die Festigkeit der mit derartigen Binden hergestellten Stützverbände den Erfordernissen der Praxis nicht gerecht wurde. Außerdem führt die hohe Reaktivität der Oxicarbonylisocyanate zu einer äußerst geringen Lagerstabilität der imprägnierten Binden, da die Oxicarbonylisocyanat- und Urethangruppen aufweisenden Präpolymere der DE-OS 2 353 212 schon in Anwesenheit von Spuren von Feuchtigkeit rasch aushärten.

Das in der DE-OS 2 357 931 beschriebene Verfahren zur Herstellung von ausgehärteten Wickelkörpern hat dem gegenüber für den medizinischen Einsatz den Nachteil, daß die in dieser Veröffentlichung beschriebenen Aushärtung durch die Einwirkung von Luftfeuchtigkeit einen relativ langen Zeitraum in Anspruch nimmt.

Schneller aushärtende Binden und deren Verwendung für medizinische Stützverbände werden in der DE-OS 2 651 089 beschrieben (= FR-A-2 369 830).

Die dort erwähnten Verbände sind meistens entweder durch die reaktive Bindenbeschichtung selbst oder durch im Beschichtungsmaterial mitverwendete Pigmente oder Farbstoffe gefärbt. Bei entsprechender Wahl der Ausgangskomponenten (z. B. gemäß Beispiel 12 der DE-OS 2 651 089) können auch farblose Binden erhalten werden, die jedoch aufgrund der in der Imprägnierung enthaltenen aromatischen Gruppen zur Vergilbung neigen. Solche Verbände werden daher schon nach kurzer Zeit unansehnlich. Wie Beispiel 16 der DE-OS 2 651 089 zeigt, führt die Verwendung von nicht vergilbendem Beschichtungsmaterial zu für den gewünschten Einsatzzweck unggeignet langen Aushärtungszeiten.

Durch die vorliegende Erfindung werden nun witterungsbeständige, nicht vergilbende Stützverbände für den medizinischen bzw. tiermedizinischen Einsatz zur Verfügung gestellt, welche nicht mehr die Nachteile der genannten Materialien des Standes der Technik aufweisen. Die erfindungsgemäßen Stützverbände haben neben den in der DE-OS 2 651 089 erwähnten Vorteilen zusätzlich den Vorteil ausreichender Stabilität gegen Witterungs- und Lichteinflüsse.

Gegenstand der vorliegenden Erfindung ist somit ein selbsthärtendes Material für die Herstellung witterungsbeständiger, nicht vergilbender Stützverbände für den medizinischen oder tiermedizinischen Einsatz, bestehend aus einem luftdurchlässigen, flexiblen Flächengebilde, welches mit 50 bis 300 Gew.-%, bezogen auf unbehandeltes Flächengebilde, eines NCO-Präpolymeren auf Basis von aromatischen Polyisocyanaten und tertiären Aminstickstoff aufweisenden Polyolen imprägniert bzw. beschichtet ist, wobei die Präpolymeren einen NCO-Gehalt von 5 bis 30 Gew.-% und einen Gehalt an tertiärem Aminstickstoff von 0,01 bis 2,5 Gew.-% aufweisen, welches dadurch gekennzeichnet ist, daß das NCO-Präpolymere 0,05 bis 3 Gew.-%, vorzugsweise 0,2 bis 2 Gew.-%, eines der nachstehenden Lichtschutzmittel enthält:

(a) substituierte Indoline der allgemeinen Formel

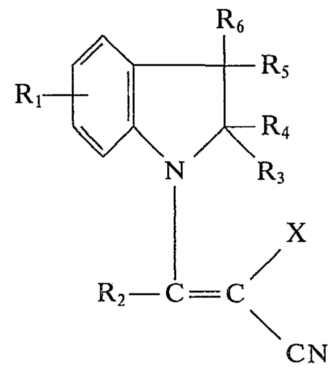

in der

$R_1$ ein Wasserstoffatom oder eine Methoxygruppe,
$R_2$ ein Wasserstoffatom oder eine Methylgruppe,
$R_3$ bis $R_6$ unabhängig voneinander ein Wasserstoffatom oder eine Methylgruppe, wobei auch $R_4$ und $R_5$ miteinander verbunden sein können, so daß ein gesättigter 6-Ring vorliegt und
X eine Cyanogruppe oder eine $-COOR_7$-Gruppe, in der $R_7$ für einen Alkylrest mit 1 bis 8 C-Atomen steht, oder eine $-CONR_8R_9$-Gruppe, in der $R_8$ und $R_9$ unabhängig voneinander für ein Wasserstoffatom oder eine Methylgruppe stehen, bedeuten,

und/oder

(b) substituierte Oxalanilide der allgemeinen Formel

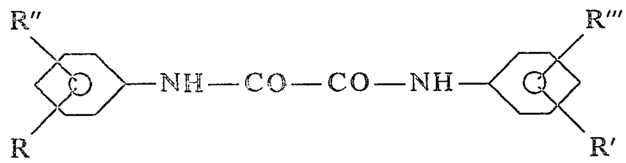

in welcher

R und R' unabhängig voneinander für einen gegebenenfalls verzweigten Alkyl- oder Alkoxyrest mit 1 bis 15, vorzugsweise 1 bis 8 C-Atomen und
R'' und R''' unabhängig voneinander für Wasserstoff oder einen gegebenenfalls verzweigten Alkyl- oder Alkoxyrest mit 1 bis 15, vorzugsweise 1 bis 8 C-Atomen stehen.

Erfindungsgemäß bevorzugt sind die unter (a) genannten Stabilisatoren, insbesondere jene, in welcher X eine Estergruppe darstellt. Die Herstellung derartiger Verbindungen wird in DE-AS 1 568 541 (US-PS 3 637 745; CH-PS 478 878) beschrieben. Es wird dort auch auf die Verwendung solcher Verbindungen als UV-Stabilisatoren für Kunststoffe, beispielsweise (Beispiel 14) Polyurethanlacke, hingewiesen. Der Literatur ist jedoch keine Anregung zu entnehmen, derartige Stabilisatoren zur Lösung der der vorliegenden Erfindung zugrunde liegenden Aufgabenstellung einzusetzen.

Bei den erfindungsgemäßen Binden handelt es sich um mit bestimmten lichtschutzmittelhaltigen Isocyanatpräpolymeren imprägnierte, luftdurchlüssige flexible Flächengebilde eines Flächengewichts von 20—10000, vorzugsweise 30—500 g/m², vorzugsweise auf textiler Basis. Geeignete derartige Flächengebilde sind beispielsweise

1. Textile Gewebe, Gewirke oder Gestricke eines Flächengewichts von 20—200 g/m², vorzugsweise 40—100 g/m² mit einer Fadenzahl von vorzugsweise 2—20 Fäden je laufendem Zentimeter in der Längs- und Querrichtung. Das textile Gewebe oder Gewirke kann aus beliebigen natürlichen oder synthetischen Garnen hergestellt sein. Vorzugsweise werden jedoch Gewebe oder Gewirke eingesetzt, die aus Mischgarnen hergestellt worden sind, die ihrerseits sowohl aus hydrophoben Fäden bzw. Fasern eines hohen E-Moduls (beispielsweise Polyester) und hydrophilen natürlichen oder synthetischen Fäden bzw. Fasern (beispielsweise Baumwolle oder Polyamid) erhalten worden sind.
2. Glasfasergewebe, -gewirke oder -gestricke eines Flächengewichts von 60—500 g/m² vorzugsweise 100—400 g/m² und einer Fadenzahl von vorzugsweise 2—20 cm in Längs- und Querrichtung. Glasfasergewebe, welche mit einer hydrophilen Schlichte versehen sind, sind bevorzugt.

3. Nicht gebundene oder gebundene oder genadelte Vliese auf Basis von anorganischen und vorzugsweise organischen Fasern eines Flächengewichts von 30—400 g/m², vorzugsweise 50—200 g/m².

Für die Herstellung von erfindungsgemäßen Versteifungsverbänden in Form von Schalen oder Schienen sind auch Vliese der obengenannten Art von Flächengewichten bis 1000 g/m² geeignet.

Die unter 1. genannten Gewebe, Gewirke oder Gestricke sind besonders bevorzugt.

Zur Imprägnierung der oben beispielhaft genannten flexiblen Flächengebilde geeignete Präpolymere sind solche, die 5—30 Gew.-%, vorzugsweise 10—25 Gew.-%, an aromatisch gebundenen Isocyanatgruppen, 0,01—2,5, vorzugsweise 0,1—1,5 Gew.-% an tertiärem Aminstickstoff und 0,05—3 Gew.-%, vorzugsweise 0,2—2 Gew.-% an Lichtschutzmittel aufweisen. Durch geeignete Auswahl der Viskosität der Ausgangsmaterialien zur Herstellung der Präpolymeren sollte darüber hinaus dafür Sorge getragen werden, daß die Präpolymere eine Viskosität von 3—50, vorzugsweise 5—30 Pa.s/25°C aufweisen.

Die Herstellung der lichtschutzmittelhaltigen Präpolymeren erfolgt in an sich bekannter Weise durch Umsetzung von überschüssigen Mengen an aromatischem Polyisocyanat mit tertiären Aminstickstoff aufweisenden Polyolen, wobei das Lichtschutzmittel zu jedem beliebigen Zeitpunkt der Herstellung zugesetzt werden kann.

Geeignete Polyisocyanate sind beliebige farblose bis schwach gefärbte aromatische Polyisocyanate, wie sie in »POLYURETHANES«, Chemistry and Technology, Teil I, Interscience Publishers (1962) oder im »Kunststoff-Handbuch«, Band VII, Polyurethane, Carl-Hanser-Verlag München (1966) beschrieben sind, beispielsweise 2,4-Diisocyanatotoluol, 2,6-Diisocyanatotoluol, aus diesen Isomeren bestehende Gemische, 4,4'-Diisocyanatodiphenylmethan, 2,4'-Diisocyanatodiphenylmethan, aus diesen Isomeren bestehende, gegebenenfalls noch geringe Mengen an 2,2'-Diisocyanatodiphenylmethan enthaltende Gemische oder beliebige Gemische der vorgenannten Polyisocyanate.

Vorzugsweise werden farblose bis schwach gefärbte Diisocyanatodiphenylmethan-Isomerengemische eingesetzt. Geeignete tertiären Aminstickstoff aufweisende Polyole sind beispielsweise

1. Tertiären Stickstoff aufweisende ethergruppenfreie Polyole des Molekulargewichtsbereichs 105—300 wie z. B. N-Methyl-diethanolamin, N-Ethyl-diethanolamin, N-Methyl-dipropanolamin, Triethanolamin oder Tripropanolamin;
2. Tertiären Stickstoff aufweisende Polyesterpolyole des Molekulargewichtsbereichs 300—4000, vorzugsweise 800—2000, wie sie durch Umsetzung von mehrbasischen Säuren mit Aminoalkoholen der oben beispielhaft genannten Art, gegebenenfalls unter gleichzeitiger Mitverwendung von Stickstoff-freien mehrwertigen Alkoholen erhalten werden können. Geeignete mehrwertige Säuren sind beispielsweise Adipinsäure, Phthalsäure oder Hexahydrophthalsäure. Geeignete Stickstoff-freie mehrwertige Alkohole zur Herstellung der Polyester sind beispielsweise Ethylenglykole, Tetramethylenglykol, Hexamethylenglykol, Neopentylglykol und Trimethylolpropan.
3. Tertiären Aminstickstoff aufweisende Polyetherpolyole des Molekulargewichtsbereichs 300—4000, vorzugsweise 800—2000, wie sie in an sich bekannter Weise durch Alkoxilierung von stickstoffhaltigen Startmolekülen erhalten werden können. Geeignete stickstoffhaltige Startmoleküle sind beispielsweise die oben beispielhaft genannten Aminoalkohole, Ammoniak oder mindestens 2 N-H-Bindungen aufweisende Amine wie z. B. Ethylendiamin, Anilin, Toluylendiamin oder Hexamethylendiamin. Geeignete Alkylenoxide zur Herstellung der Polyether sind beispielsweise Ethylenoxid oder Propylenoxid. Besonders bevorzugt werden die Propoxilierungsprodukte der genannten Stickstoff-haltigen Startermoleküle eingesetzt.

Aus den DE-Offenlegungsschriften 2 737 670 und 2 737 671 ist es bekannt, die Beständigkeit spezieller Polyurethanschaumstoffe gegen Witterungs- und Lichteinflüsse durch bestimmte Lichtschutzmittel zu erreichen (siehe in diesem Zusammenhang auch »Die Stabilisierung der Kunststoffe gegen Licht und Wärme«, J. Voigt, Springer Verlag (1966)). Derartige an sich bekannte Lichtschutzmittel für Polyurethanschaumstoffe sind z. B.:

I) Piperidinderivate

4-Benzoyloxy-, 4-Salicyloyloxy-, 4-Capryloxy-, 4-Stearoyloxy-,
4-(β-3,5-Di-tert.-butyl-4-hydroxyphenyl-propionyloxy-,
4-(3,5-Di-tert.-butyl-4-hydroxybenzoxy)-2,2,6,6-tetramethylpiperidin;
4-Benzoyloxy-, 4-Salicyloyloxy-, 4-Stearoyloxy- und
4-tert.-Butylbenzoyl-1,2,2,6,6-pentamethylpiperidin;
Bis-(2,2,6,6-tetramethyl-4-piperidyl)-sebacat,
Bis-(2,2,6,6-tetramethyl-4-piperidyl)-suberat,
Bis-(2,2,6,6-tetramethyl-4-piperidyl)-dodecandioat,

Bis-(1,2,2,6,6-pentamethyl-4-piperidyl)-sebacat;
4-Capryloyloxy-1-propyl-2,2,6,6-tetramethylpiperidin;
4-Capryloyloxy-1-allyl-2,2,6,6-tetramethylpiperidin;
4-Benzoylamido-, 4-Acryloxyamido- und
4-Stearoylamido-2,2,6,6-tetramethylpiperidin.

2,4,6-Tris-(2,2,6,6-tetramthyl-4-piperidyloxy)-s-triazin,
2,4,6-Tris-(1,2,2,6,6-pentamethyl-4-piperidyloxy)-s-triazin,
2,4,6-Tris-(2,2,6,6-tetramethyl-4-piperidylamino)-s-triazin,
2,2,6,6-Tetramethyl-4-β-cyanoethoxy-piperidin,
1,2,2,6,6-Pentamethyl-4-lauroyloxy-piperidin und
Triacetonaminoxim.

II) Benzophenonderivate

2,4-Dihydroxy-, 2-Hydroxy-4-methoxy-, 2-Hydroxy-4-octoxy-,
2-Hydroxy-4-dodecyloxy-, 2-Hydroxy-4-benzyloxy-,
2-Hydroxy-4,4′-dimethoxy-, 2,4,4′-Trihydroxy-,
2,2′-Dihydroxy-4,4′-dimethoxy-, 2,2′,4,4-Tetrahydroxy-,
2,2′-Dihydroxy-4-methoxy-, 2-Hydroxy-2′-carboxy-4-methoxy-,
2,2′-Dihydroxy-4-octoxy- und
2,2′-Dihydroxy-4-dodecyloxy-benzophenon.

III) Benztriazolderivate

2-(2′-Hydroxy-5′-methylphenyl)-, 2-(2′-Hydroxy-5′-tert.-butylphenyl)-,
2-(2′-Hydroxy-5′-tert.-octylphenyl)-, 2-(2′-Hydroxy-3′-tert.-butyl-5′-methylphenyl)-,
2-(2′-Hydroxy-3′-tert.-butyl-5′-methylphenyl)-5-chlor-,
2-(2′-Hydroxy-3′,5′-di-tert.-butylphenyl)-,
2-(2′-Hydroxy-3′,5′-di-tert.-butylphenyl)-5-chlor-,
2-(2′-Hydroxy-3′,5′-di-tert.-amylphenyl)-,
2-(2′-Hydroxy-3′,5′-di-tert.-amylphenyl)-5-chlor-,
2-(2′-Hydroxy-3′-sec.-butyl-5′-tert.-butylphenyl)-,
2-(2′-Hydroxy-3′-tert.-butyl-5′-sec.-butylphenyl)-,
2-(2′,4′-Dihydroxyphenyl)-, 2-(2′-Hydroxy-4′-methoxyphenyl)-,
2-(2′-Hydroxy-4′-octoxyphenyl)-, 2-(2′-Hydroxy-3′-α-phenylethyl-5′-methylphenyl)-
und
2-(2′-Hydroxy-3′-α-phenylethyl-5′-methyl-phenyl)-5-chlor-benztriazol.

IV) Oxalanilide (erfindungsgemäß einsetzbar)

2-Ethyl-2′-ethoxy-, 2-Ethoxy-2′-ethoxy-5′-tert.-butyl-,
2-Ethyl-4-tert.-butyl-2′-ethoxy-5′-tert.-butyl-, 2,2′-Dimethoxy-,
2,2′-Diethoxy-, 2,2′-Di-tert.-butyl-, 4,4′-Dimethoxy-, 4,4′-Diethoxy-,
2,4′-Dimethoxy-, 2,4′-Diethoxy-, 2-Methoxy-2′-ethoxy-, 2-Methoxy-4′-ethoxy-,
2-Ethoxy-4′-methoxy-, 2,2′-Dioctoxy-5,5′-di-tert.-butyl-,
2,2′-Didodecyloxy-5,5′-di-tert-butyl-, 2-Ethyl-2′-octoxy-,
4,4′-Di-octoxy-, 2-Ethyl-2′-butoxy- und 4-Methyl-4′-methoxy-oxalanilid.

V) Salicylsäurephenylester und -derivate

Salicylsäurephenylester, Salicylsäure-4-tert.-butylphenylester,
Salicylsäure-4-tert.-octylphenylester.

VI) Zimtsäureesterderivate

α-Cyano-β-methyl-4-methoxyzimtsäuremethylester,
α-Cyano-β-methyl-4-methoxyzimtsäurebutylester,
α-Cyano-β-phenyl-zimtsäureethylester,
α-Cyano-β-phenylzimtsäureisooctylester.

VII) Malonesterderivate

4-Methoxy-benzylidenmalonsäuredimethylester,
4-Methoxy-benzylidenmalonsäurediethylester,
4-Butoxy-benzyliden-malonsäuredimethylester.

VIII) Indol- und Indolinderivate (erfindungsgemäß einsetzbar)

2-Phenylindol und
2-Methyl-1-[1-cyano-1-methoxycarbonyl-ethen-(1)-yl-(2)]-indolin.

Überraschenderweise wurde jedoch gefunden, daß der gewünschte stabilisierende Effekt bei den erfindungsgemäßen Stützverbänden nur bei Verwendung von Lichtschutzmitteln der oben angegebenen allgemeinen Formeln (entsprechend den Substanzklassen IV und VIII) in befriedigender Weise erreicht wird.

Die Imprägnierung der erfindungsgemäßen Binden mit den beispielhaft genannten lichtschutzmittelhaltigen Präpolymeren kann auf beliebige Art erfolgen. Hierbei werden die Binden beispielsweise einem Tränk- oder Beschichtungsprozeß auf konventionellen Geräten etwa durch Rakeln, Tränken und anschließendes Abquetschen auf Walzen bzw. Abzentrifugieren, durch Besprühen oder Auftrag durch Umkehrverfahren unterworfen. Dabei kann das Präpolymere entweder in Substanz oder als Lösung zum Einsatz gelangen. Bei der Verwendung von Lösungen werden vorzugsweise leicht flüchtige Lösungsmittel wie z. B. Methylenchlorid verwendet.

Durch geeignete Auswahl des Flächengewichtes, der Maschenweite des flexiblen Trägers und der Auftragsmenge des einzusetzenden lichtschutzmittelhaltigen Präpolymeren innerhalb der oben angegebenen Bereiche wird dafür Sorge getragen, daß lediglich eine Ummantelung der Fasermaterialien unter Aufrechterhaltung von Hohlräumen zwecks Gewährleistung der gewünschten Atmungsaktivität erfolgt.

Das imprägnierte Trägermaterial wird im Falle der Mitverwendung von Hilfslösungsmitteln von diesen beispielsweise durch eine Vakuumbehandlung befreit. Nach der Imprägnierung können die erhaltenen erfindungsgemäßen Binden in geschlossenen Vorrichtungen in Abwesenheit von Feuchtigkeit gelagert werden. Vorzugsweise erfolgt die Lagerung der erfindungsgemäßen Binden in Form von Wickelkörpern oder in getafelter Form in luftdicht verschlossenen Behältern aus Metallen wie beispielsweise Aluminium. Besonders geeignet zur Lagerung der erfindungsgemäßen Binden sind verschweißte Beutel aus Polyethylen-beschichteten Aluminiumfolien oder feuchtigkeitsdicht verschlossene Aluminiumdosen. Die Binden können dann zu einem beliebigen Zeitpunkt aus dem Behälter entnommen werden.

Bei der Applikation der erfindungsgemäßen Binden wird der zu stützende Körperteil zunächst mit einer luftdurchlässigen, nicht imprägnierten Unterfütterung abgedeckt. Geeignete Materialien für diese Unterfütterung sind beispielsweise offenporiges Papier, Vliesstoffe oder textile Bindenmaterialien. Vorzugsweise werden solche Materialien zur Unterfütterung eingesetzt, die nur eine beschränkte Hydrophilie aufweisen. Besonders gut geeignet ist daher beispielsweise ein Vliesmaterial aus Polyester oder Polypropylen.

Auf das so unterfütterte Körperteil wird anschließend eine vorher mit Wasser gesättigte erfindungsgemäße Binde aufgewickelt. Die Wassersättigung der erfindungsgemäß einzusetzenden Binden erfolgt unmittelbar vor deren Anwendung, beispielsweise durch Eintauchen der Binden in Wasser.

Die Dicke des so hergestellten Stützverbandes richtet sich nach den jeweiligen medizinischen Anforderungen. Sie liegt im allgemeinen zwischen 3 und 10 mm. Die erfindungsgemäßen Binden können sowohl zur Herstellung von Stützverbänden durch Umwicklung der zu stützenden Körperteile als auch, gegebenenfalls in getafelter Form, zur Herstellung von Schalen oder Schienen eingesetzt werden.

Die erfindungsgemäßen witterungsbeständigen, nicht vergilbten Stützverbände zeichnen sich durch hervorragende mechanische Eigenschaften aus, insbesondere durch ein ausgewogenes Verhältnis von Steifigkeit und Elastizität.

Die nachfolgenden Beispiele dienen zur Erläuterung der Erfindung. Wenn nicht anders vermerkt, sind Mengenangaben als Gewichtsteile bzw. Gewichtsprozente zu verstehen.

## Beispiele 1—5

12 cm breite Verbandmullbinden aus einem Mischgarn von 40% Baumwolle und 60% Viskose, einem Flächengewicht von 30 g/m$^2$ und einer Fadenzahl von 12 Fäden/cm in der Kette und 8 Fäden/cm im Schuß wurden unter Feuchtigkeitsausschluß jeweils mit 25 g eines lichtschutzmittelhaltigen Präpolymeren (Art und Menge des Lichtschutzmittels siehe Tabelle) aus einem Diisocyanatodiphenylmethan-Isomerengemisch (Gewichtsverhältnis 4,4'-: 2,4'-Isomer = 1 : 1,5) und propoxyliertem Triethanolamin (OH-Zahl 148) im Gewichtsverhältnis 1,25 : 1 (12 Gew.-% NCO-Gruppen, 0,5 Gew.-% tert.-Aminstickstoff), welches als Lösung in Methylenchlorid vorlag (Gewichtsverhältnis Präpolymer zu Lösungsmittel = 1 : 1), imprägniert. Nach der Imprägnierung wurde das Lösungsmittel im Ölpumpenvakuum entfernt und die imprägnierten Binden auf einen Polyethylenkern gewickelt oder getafelt und in einem Siegelrandbeutel aus Dreischichtenlaminat Polyester/Aluminium/Polyethylen versiegelt.

Nach einer Lagerzeit von 1 Woche bei ca. 25°C wurden die imprägnierten Binden aus der Verpackung genommen, für 3—5 Sekunden in Wasser von ca. 25°C getaucht und leicht geknetet. Anschließend wurden innerhalb von 3 Minuten röhrenförmige Formkörper von 42 mm Innendurchmesser und ca. 12 cm Länge gewickelt.

Unter leichter Wärmeentwicklung (Oberflächentemperatur max. 35°C) härteten die Stützverbände in weiteren 5 Minuten zu stabilen, belastungsfähigen Formkörpern mit hervorragender Verbundhaftung aus.

Die ausgehärteten Stützverbände wurden mit jeweils 2 in einem Abstand von 25 cm zur Probe und 2 in einem Abstand von 12 cm zur Probe angeordneten Schwarzlichtlampen des Typs TL 20 W/08 der Firma Philips (Wellenlängenbereich 300—400 mm, Intensitätsmaximum 350 mm) 50 Stunden (mit Normlichtart C) bestrahlt. Die Vergilbung der Proben wurde nach DIN 5033 über die Helmholtz-Maßzahlen gemessen.

Der Vergilbungsgrad ist aus der Tabelle ersichtlich.

Folgende Lichtschutzmittel wurden untersucht:

Beispiel 1: 2-Methyl-1-[1-cyano-1-methoxycarbonylethen-(1)-yl-(2)]-indolin
Beispiel 2: 2,2'-Di-tert.-butyloxalanilid
Beispiel 3: (Vergleich) 2 (n-Butyl)-2[3,5-di-tert.-butyl)-benzyl-]malonsäure-di-(1,2,2,6,6-pentamethyl-4-piperidyl)-ester
Beispiel 4: (Vergleich) Bis-(2,2,6,6-tetramethyl-4-piperidyl)-sebacat
Beispiel 5: (Vergleich) 2-(2'-Hydroxy-5'-methylphenyl)-benztriazol

In der Tabelle bedeuten die unter »optischer Eindruck« angegebenen Zahlen:

1 keine Vergilbung erkennbar
2 sehr schwache Vergilbung
3 geringe Vergilbung
4 merklich vergilbt
5 stark vergilbt

Tabelle

| Beispiel | Konzentration des Lichtschutzmittels (%) | Helmholtz-Maßzahl Pc Bestrahlung vor | nach | $\Delta$Pc | Optischer Eindruck |
|---|---|---|---|---|---|
| Nullversuch | 0 | 0,073 | 0,46 | 0,39 | 5 |
| 1a | 0,25 | 0,070 | 0,29 | 0,22 | 1 |
| 1b | 1 | 0,064 | 0,18 | 0,12 | |
| 2a | 0,25 | 0,098 | 0,36 | 0,26 | 2 |
| 2b | 1 | 0,12 | 0,34 | 0,22 | |
| 3a | 0,25 | 0,071 | 0,43 | 0,36 | 4 |
| 3b | 1 | 0,11 | 0,40 | 0,29 | |
| 4 | 1 | 0,085 | 0,45 | 0,37 | 5 |
| 5 | 1 | 0,085 | 0,52 | 0,44 | 5 |

**Patentansprüche**

1. Selbsthärtendes Material für die Herstellung witterungsbeständiger, nicht vergilbender Stützverbände für den medizinischen oder tiermedizinischen Einsatz, bestehend aus einem luftdurchlässigen, flexiblen Flächengebilde, welches mit 50 bis 300 Gew.-%, bezogen auf unbehandeltes Flächengebilde, eines NCO-Präpolymeren auf Basis von aromatischen Polyisocyanaten und tertiären Aminstickstoff aufweisenden Polyolen imprägniert bzw. beschichtet ist, wobei die Präpolymeren einen NCO-Gehalt von 5 bis 30 Gew.-% und einen Gehalt an tertiärem Aminstickstoff von 0,01 bis 2,5 Gew.-% aufweisen, dadurch gekennzeichnet, daß das NCO-Präpolymer 0,05 bis 3 Gew.-% (bezogen auf Präpolymer) an

(a) substituierten Indolinen der allgemeinen Formel

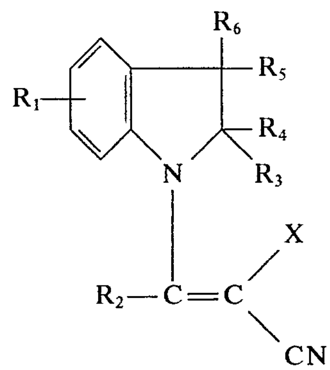

in der

$R_1$ ein Wasserstoffatom oder eine Methoxygruppe,
$R_2$ ein Wasserstoffatom oder eine Methylgruppe,
$R_3$ bis $R_6$ unabhängig voneinander ein Wasserstoffatom oder eine Methylgruppe, wobei auch $R_4$ und $R_5$ miteinander verbunden sein können, so daß ein gesättigter 6-Ring vorliegt und
X eine Cyanogruppe oder eine $-COOR_7$-Gruppe, in der $R_7$ für einen Alkylrest mit 1 bis 8 C-Atomen steht, oder eine $-CONR_8R_9$-Gruppe, in der $R_8$ und $R_9$ unabhängig voneinander für ein Wasserstoffatom oder eine Methylgruppe stehen, bedeuten,

und/oder

(b) substituierten Oxalaniliden der allgemeinen Formel

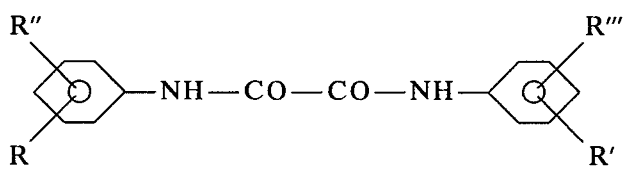

in welcher

R und R′ unabhängig voneinander für einen gegebenenfalls verzweigten Alkyl- oder Alkoxyrest mit 1 bis 15 C-Atomen und
R″ und R‴ unabhängig voneinander für Wasserstoff oder einen gegebenenfalls verzweigten Alkyl- oder Alkoxyrest mit 1 bis 15 C-Atomen stehen,

als Lichtschutzmittel enthält.

2. Material nach Anspruch 1, dadurch gekennzeichnet, daß das Lichtschutzmittel der Formel (a) entspricht, in welcher X für eine Estergruppe steht.

3. Material nach Anspruch 1, dadurch gekennzeichnet, daß das Lichtschutzmittel 2-Methyl-1-[1-cyano-1-methoxy-carbonylethen-(1)-yl-(2)]-indolin ist.

**Claims**

1. A self-curing material for the production of weather-resistant, non-yellowing support bandages for medical or veterinary use, comprising an air-permeable, flexible sheet which is impregnated or coated with from 50 to 300%, by weight, based in untreated sheet, of a NCO-prepolymer based on aromatic polyisocyanates and polyols containing tertiary amine nitrogen, wherein the prepolymers have a NCO content of from 5 to 30%, by weight, and a content of tertiary amine nitrogen of from 0.01 to 2.5%, by weight, characterised in that the NCO-prepolymer contains from 0.05 to 3%, by weight, (bases on prepolymer) of:

(a) substituted indolines of the general formula

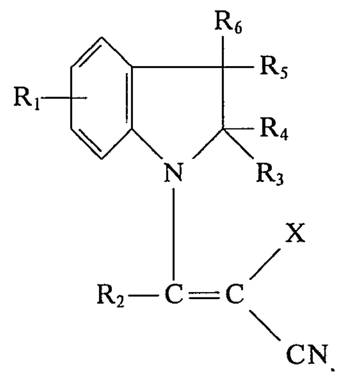

wherein

- $R_1$ represents a hydrogen atom or a methoxy group;
- $R_2$ represents a hydrogen atom or a methyl group;
- $R_3$ to $R_6$ each independently represents a hydrogen atom or a methyl group; or $R_4$ and $R_5$ may be conjoint giving a saturated 6 membered ring; and
- X represents a cyano group or a $-COOR_7$ group wherein $R_7$ represents an alkyl radical having from 1 to 8 carbon atoms; or a $-CONR_8R_9$ group wherein $R_8$ and $R_9$ each independently represents a hydrogen atom or a methyl group;

and/or

(b) substituted oxalanilides of the general formula

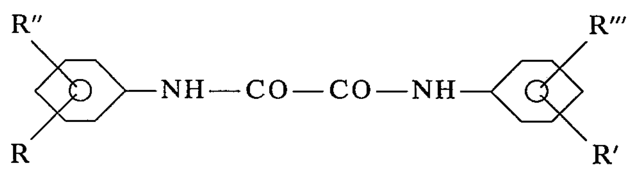

wherein

- R and R′ each independently represents an optionally branched alkyl or alkoxy radical having from 1 to 15 carbon atoms; and
- R″ and R‴ each independently represents hydrogen or an optionally branched alkyl or alkoxy radical having from 1 to 15 carbon atoms;

as light protection agent.

2. A material according to claim 1, characterised in that the light protection agent corresponds to formula (a) wherein X represents an ester group.

3. A material according to claim 1, characterised in that the light protection agent is 2-methyl-1-[1-cyano-1-methoxy-carbonylethene-(1)-yl-(2)]-indoline.

**Revendications**

1. Matière autodurcissante pour la formation de pansements de soutien ne jaunissant pas, résistant aux agents atmosphériques et destinés à être utilisés en médecine humaine ou vétérinaire, cette matière étant constituée d'un article superficiel flexible perméable à l'air, imprégné ou enduit de 50 à

300% en poids (calculé sur l'article superficiel non traité) d'un prépolymère de NCO à base de polyisocyanates aromatiques et de polyols comportant de l'azote d'amine tertiaire, les prépolymères ayant une teneur en groupes NCO de 5 à 30% en poids et une teneur en azote d'amine tertiaire de 0,01 à 2,5% en poids, caractérisée en ce que, comme agent de protection contre la lumière, le prépolymere de NCO contient 0,05 à 3% en poids (calculé sur le prépolymère) des substances suivantes:

(a) des indolines substituées de formule générale:

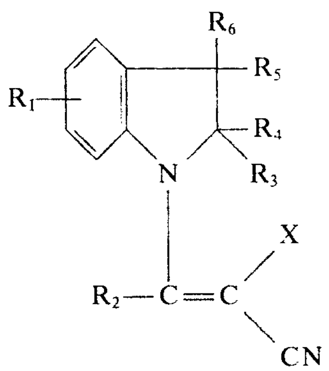

dans laquelle

| | |
|---|---|
| $R_1$ | représente un atome d'hydrogène ou un groupe méthoxy, |
| $R_2$ | représente un atome d'hydrogène ou un groupe méthyle, |
| $R_3$ à $R_6$ | représentent indépendamment l'un de l'autre un atome d'hydrogène ou un groupe méthyle, $R_4$ et $R_5$ pouvant également être reliés l'un à l'autre de telle sorte que l'on ait un noyau hexagonal saturé, et |
| X | représente un groupe cyano ou un groupe $-COOR_7$ dans lequel $R_7$ représente un groupe alkyle contenant 1 à 8 atomes de carbone, ou un groupe $-CONR_8R_9$ dans lequel $R_8$ et $R_9$ représentent indépendamment l'un de l'autre un atome d'hydrogène ou un groupe méthyle, |

et/ou

(b) des oxalanilides substitués de formule générale:

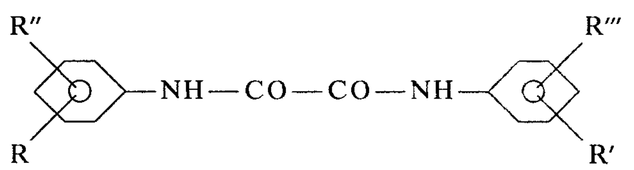

dans laquelle

| | |
|---|---|
| R et R′ | représentent indépendamment l'un de l'autre un groupe alkyle ou alcoxy éventuellement ramifié et contenant 1 à 15 atomes de carbone, et |
| R″ et R‴ | représentent indépendamment l'un de l'autre un atome d'hydrogène ou un groupe alkyle ou alcoxy éventuellement ramifié contenant 1 à 15 atomes de carbone. |

2. Matière suivant la revendication 1, caractérisée en ce que l'agent de protection contre la lumière répond à la formule (a) dans laquelle X représente un groupe ester.

3. Matière suivant la revendication 1, caractérisée en ce que l'agent de protection contre la lumière est la 2-méthyl-1-[1-cyano-1-méthoxy-carbonyléthén-(1)-yl-(2)]-indoline.